**Europäisches Patentamt**

(19) **European Patent Office**

**Office européen des brevets**

(11) Publication number : **0 354 543 B1**

(12) # EUROPEAN PATENT SPECIFICATION

(45) Date of publication of patent specification :
**11.11.92 Bulletin 92/46**

(51) Int. Cl.⁵ : **A61K 49/00, A61K 49/02, A61K 43/00, A61K 39/395**

(21) Application number : **89114680.5**

(22) Date of filing : **08.08.89**

(54) **Photochemical attachment of chelating groups to biomolecules.**

(30) Priority : **08.08.88 US 229672**

(43) Date of publication of application :
**14.02.90 Bulletin 90/07**

(45) Publication of the grant of the patent :
**11.11.92 Bulletin 92/46**

(84) Designated Contracting States :
**AT BE CH DE ES FR GB GR IT LI LU NL SE**

(56) References cited :
**EP-B- 0 127 438**
**GB-A- 2 137 612**
**US-A- 3 843 447**
**US-A- 4 689 310**
**US-A- 4 716 122**

(73) Proprietor : **BIOMIRA, INC.**
**Research Centre One Edmonton R&D Park**
**9411A 20th Avenue**
**Edmonton Alberta T6N 1E5 (CA)**

(72) Inventor : **Noujaim, Antoine A.**
**78 Willow Way**
**Edmonton Alberta T5T 1C8 (CA)**
Inventor : **Gopalakrishnan, Geetha**
**No.206, 12325 Landsdowne Drive**
**Edmonton Alberta T6H 4L4 (CA)**
Inventor : **Sykes, Thomas Ritchie**
**4123 Ramsay Road**
**Edmonton Alberta T6H 5L5 (CA)**

(74) Representative : **Patentanwälte Grünecker,**
**Kinkeldey, Stockmair & Partner**
**Maximilianstrasse 58**
**W-8000 München 22 (DE)**

EP 0 354 543 B1

Jouve, 18, rue Saint-Denis, 75001 PARIS

**Description**

Conjugates of chelating agents and biomolecules are known. Lee, EP Appl. 173,629 (Cytogen); Rodwell, U.S. 4,671,958; Stavrianopoulos, U.S. 4,707,352; Palk, U.S. 4,652,440; Gansow, WO86/006384.

Bifunctional chelating agents (BFCAs) have been used to associate a chelatable ion with a biomolecule. One functionality of the BFCA chelates the ion while the other couples the biomolecule. The resulting conjugates are of value as diagnostic and therapeutic agents. For example, radiolabelled proteins such as human serum albumin (Hnatowich, et al., Int. J. Appl. Radiat. Iso., 33: 327 [1982]) and fibrinogen (Layne, et al., J. Nucl. Med., 23: 627 [1982]) have been used as radiopharmaceuticals for blood volume determination and clot localization, respectively, in patients. M.W. Brechbiel, et al., (Inorg. Chem. 25: 2772 [1986]) reports the use of a MoAB B72.3-chelator conjugate in clinical trials for diagnosis of human colorectal cancer and there are certainly many others. (Biochem. Biophys. Acta. 780:151 [1985]).

The amino function ($-NH_2$) of amino-Benzyl-EDTA may be converted to a diazo group ($-N_2^+$) (Sundberg, et al., J. Med. Chem., 17: 1304 [1974]), or to an isothiocyanate (-NCS) or bromoacetamido ($-NHCOCH_2Br$) function (Meares, et al., Anal. Biochem., 142: 68 [1984])), for reaction with a protein. In DTPA, the $-CH_2N-(CH_2COOH)_2$ has been modified to bear a dianhydride (Hnatowich, et al., J. Immunol. Methods, 65: 147 [1983]), a carboxycarbonic anhydride (Krecjarek, et al., Biochem. Biophys. Res. Commun., 77: 581 [1977]), a succinimide (Najafi, et al., Nucl. Med. Biol., 13: 345 [1986]) or a benzyl isothiocyanate for the same purpose.

Sundoro, U.S. 4,680,338, is a typical patent on a bifunctional linker suitable for conjugating a ligand (e.g., a chelator) with an amine-containing protein.

The overall protocol for the use of BFCAs usually takes two forms. The protein may be 'pre-chelated' in which case the BFCA is attached to the protein first and the metal ion added to the purified (to remove unbound BFCA) protein. Alternately, the BFCA may be allowed to first bind the metal ion and then conjugated to the protein. In this case final purification removes unbound BFCA and unbound metal ion. In practical terms, both approaches are useful depending on the requirements. The preparation of a pre-chelated protein is desirable when large identical batches are to be prepared, when radioactive metal ions are used, and when final metal ion binding is highly efficient. Pre-chelation of the metal ion usually enables quantitative formation of the metal chelate since both reactants may be kept at high concentration and additional reagents to effect the process may be used (e.g., stannous ion reduction of pertechnetate in the presence of BFCA). However, unless the BFCA to protein attachment is a high yielding reaction, most of the potentially expensive metal ion (e.g., radioindium) may be wasted.

The currently useful protein-reactive groups on BFCAs (e.g., diazo, isothiocyanate, bromoacetamide, anhydride, succinimide and benzimidate) react with polyamine acids primarily via electrophilic attack on the amino acids cysteine, lysine, tyrosine and tryptophan.

For example in GB-A-2137612 metal chelate complexes are disclosed having a chelated group attached to a biomolecule, which are prepared by reacting activated derivatives of the complex-forming acid, for example, acid chlorides, acid anhydrides, activated esters, nitrenes or isothiocyanates with nucleophilic groups of the biomolecule, such as amino, hydroxy, thio or imidazole groups.

This fact limits the theoretical maximum number of chelates attainable on a protein given its fixed amino acid composition. This may be a problem when a high metal ion content is required to increase the sensitivity of detection (e.g., when high specific radioactivity probes are required) or to improve the kinetics and efficiency of metal ion binding (e.g., for short-lived radioactive metal ions or when labelling yields are poor). In a general sense, these BFCA may only be used to label biomolecules which possess specific chemical functionalities and several modifications of the same BFCA may be required to suit a variety of different biomolecules.

In contrast, the photoactivated BFCAs prepared according to the method of the present invention are capable of considerably higher levels of chelate attachment to proteins and to other biomolecules. The use of a photoactivated reactive group to attach a chelating agent to a biomolecule has not previously been reported.

Photoactivatable agents, including arylazides, have been used to immobilize an antigen or antibody on a support. See Kramer, U.S. 4,689,310; Scheebers, U.S. 4,716,122; AU-A-47690/85 (Organogen). Dattagupta, U.S. 4,713,326 photochemically coupled a nucleic acid to a substrate. Other molecules have also been insolubilized by this technique. Lingwood, U.S. 4,597,999. Pandey, et al., J. Immunol. Meth., 94: 237-47 (1986) conjugated a haptenic primary aromatic amine (3-azido-N-ethylcarbazole) to various proteins to create a synthetic antigen.

Furthermore, one may prepare a "prodrug" or "protoxin" which is converted to the active drug or toxin by photochemical cleavage of a bridging group. Zweig, U.S. 4,202,323; Senter, U.S. 4,625,014; Edelson, U.S. 4,612,007; Reinherz, U.S. 4,443,427 (col. 4).

Photoaffinity labeling of enzymes is also known. Chowdry, Ann. Rev. Biochem., 48: 293-305 (1979). In this technique, a probe compound is allowed to interact with a biomatrix in which a specific receptor or binding site is envisaged. Upon formation of the specific complex by their affinity for one another the pair are chemically linked via a reactive group on the probe. This greatly facilitates the identification and characterization of the receptor itself. The great advantage of probes incorporating a photoactive group is that the linkage process may be activated after all non-specific interactions of the probe have been eliminated and thus little or no indiscriminate binding or loss of probe through hydrolysis takes place. These photoaffinity reagents may be prepared either via synthetic routes for simple molecules or via conjugation of a photoactive ligand for more complex macromolecules.

It is the object of the present invention to provide a composition comprising a compound comprising a chelating group and a group which, when photoactivated, becomes highly reactive, i.e. which is capable of insertion into carbon-hydrogen nitrogen-hydrogen or oxygen-hydrogen bonds, and a method of photochemically attaching chelating groups to biomolecules.

The present invention provides a composition comprising a compound having a chelating group and a photoactivatable group which upon photoactivation is capable of reaction with a carbon-hydrogen, nitrogen-hydrogen or oxygen-hydrogen bond and a method of conjugating a chelatable ion with a biomolecule which comprises in any order the steps of (a) photoactivating the above compound in the presence of a biomolecule, whereby the photoactivated group is coupled to the biomolecule, and (b) incubating a chelatable ion with said compound under chelating conditions.

The photoactivatable bifunctional chelating agents (Ph-BFCA) represent a new class of chemical compounds having considerable advantages as means for labeling biomolecules with a chelatable ion:

1. They are amenable to labelling all types of biomolecules. The attachment mode, unique to photoactive species, requires merely a simple carbon-hydrogen, oxygen-hydrogen or nitrogen-hydrogen bond and the ubiquitous presence of these structures in virtually all biomolecules ensures universal substrates.

2. The conjugates' yield can be quantitative. This permits direct use of the chelated product without a secondary procedure for removal of unbound chelate. This eliminates a time-consuming and problematic step and makes efficient use of what could be an expensive reagent.

3. The degree of conjugation is readily controlled, generally independent of the biomolecule, and potentially unlimited. If the yield is quantitative, the number of chelating groups attached becomes a simple function of the initial reactant ratios. Thus, if the attachment of 10 chelates is desirable, the initial reagent to biomolecule ratio should be 10. Given the non-specific labelling process, this feature could be applied to any biomolecule. The number of chelates attached may be well in excess of those attainable with chemical-group specific reactions. For example, a biomolecule which contains 10 free amine groups could be not conjugated further using a amine requiring reagent, as opposed to the unlimited potential with the new class of reagents. The introduction of less bulky photoactive groups such as an azido or diazo substituent is better in steric terms (Bayley, et al., Methods Enzymol., 46: 69) [1977]).

4. The coupling is relatively mild and rapid compared to many chemical conjugation methods. Simple dissolution in a stabilizing buffer of choice at slightly basic pH and short-term irradiation (<10 minutes) with a suitable wavelength and intensity light source is all that is required. These conditions would be expected to produce minimal biological damage which is important for structure-function dependent biomolecules such as enzymes or antibodies.

The PF-BFCA compounds of the present invention have the structure

Ch - optional spacer - PF

Where Ch is an ion chelating group and PF is a photoactivatable functionality such as an azido group.

The photoactivated reactions of a PF-BFCA with biomolecules constitutes a mild, highly efficient method of introducing chelating functionalities into any biomolecule. The relatively non-selective labelling pattern of these reagents should enable molecules, which were previously difficult or impossible to label with specific metal ions, to be studied in this fashion. Substances such as oligosaccharides, mucins, lipids and individual proteins which contain few suitable sites for chemically specific attachment are now suitable substrates. Other biologically active materials such as drugs, dextran, polyethylene glycols may now be more effectively utilized as carriers of metal ion probes. Metal-ion containing biomolecules would be extremely useful as NMR contrast agents (e.g., incorporating Gd or Mn) and as fluorescent markers (e.g., incorporating Eu or Tb). The ability to label two or more biomolecules, each with a different metal-ion, allows them to be utilized in assay procedures (e.g., RIA or IRMA) to detect multiple species in a single sample. Since there is a vast supply of radioactive metal ions, the chelated biomolecules can be radiolabelled for all types of in vitro and in vivo applications. For nuclear medicine imaging desirable radioisotopes such as $^{67}$Ga, $^{111}$In or $^{99m}$Tc can be more effectively used as can those for specific procedures such as PET (e.g., $^{68}$Ga) and radiopharmaceutical therapy (e.g., $^{90}$Y).

Chelatable Ions and Chelating Groups

The present invention is not limited to any particular chelating agent. While, EDTA and DTPA derivatives are preferred, many chelating agents are known. See Meares, U.S. 4,678,667; Wieder, u.s. 4,352,751; Hnatowich, U.S. 4,479,930; Meares, U.S. 4,043,998; Ueda, U.S. 4,564,742; Davidson, U.S. 4,673,562; Hnatowich, U.S. 4,668,503, Arano, U.S. 4,559,221 and Costa, U.S. 3,809,632. The following table shows ions chelated by various agents:

| Chelating Groups | Chelatable Ions |
|---|---|
| EDTA | $^{111}In$, $^{67}Ga$, $^{99m}Tc$, $^{90}Y$ |
| DTPA | $^{99m}Tc$, $^{111}In$, $^{67}Ga$, $^{90}Y$, Gd |
| MA-DTPA | $^{111}In$, $^{67}Ga$, $^{99m}Tc$, $^{90}Y$, Gd |
| CA-DTPA | $^{111}In$, $^{67}Ga$, $^{99m}Tc$, $^{90}Y$, Gd |
| TETA;DOTA | $^{111}In$, $^{67}Ga$, $^{90}Y$ |
| DADS | $^{99m}Tc$, $^{186}Re$, $^{188}Re$ |

Photoactivable Functionality

The photoactivatable functionality (PF) is a chemical structure which, upon irradiation with light, is converted into a more reactive chemical species, (PF*) which is capable of reacting with all or most of the naturally occurring amino acids and other chemical moieties. Preferably, PF is of low chemical reactivity (in the dark) and PF* of high chemical reactivity.

In a first embodiment, the PF* is a carbene. Carbenes are typically generated by cleavage of carbon-nitrogen double bonds and are exceptionally reactive. Carbenes react very rapidly with a variety of chemical functions: by coordination to nucleophilic centers (to give carbanions), by addition to multiple bonds (including those of aromatic systems), by insertion into single bonds (including carbon-hydrogen bonds), and by hydrogen abstraction (to give two free radicals that may then couple). However, if there is a hydrogen atom on the carbon atom adjacent to the carbene center, hydrogen migration readily occurs, and results in unreactive olefin. For this reason the adjacent atom must bear no hydrogen.

Also, alpha-ketocarbenes may undergo intramolecular Wolff rearrangement, resulting in a ketene.

See "Carbenes" (M. Jones, Jr. and R.A. Moss, eds.). Wiley (Interscience), New York, Vol. I, 1973; Vol. II, 1975.

Preferably, carbenes are prepared from aryldiazirines (e.g., Aryl-$CHN_2$) or from fluorinated diazo compounds (e.g., $RC(O)(CF_3)=N^+=N^-$). These precursors are preferred because they are stable in the dark and less susceptible to rearrangement.

In a second embodiment, the PF* is a nitrene. Nitrenes are produced by photolysis of nitrogen-nitrogen bonds, such as those found in an azide. Preferably, arylnitrenes are generated by photoactivation of an arylazide. Arylazides are less chemically reactive than are acyl-, sulfonyl- or phosphorylazides, and are easily prepared. Arylnitrenes are highly reactive, uncharged, electron-deficient species. The reactions available to a nitrene are broadly similar to those undergone by carbenes, but the reactivity of nitrenes is considerably lower and hence more selective. For example, nitrenes are somewhat electrophilic, preferring an O-H over a C-H bond. The possible fates of nitrenes include addition to a double bond to yield a heterocyclic ring, nucleophilic attack, abstraction of hydrogen, and insertion into a C-H bond.

If a nitrene is generated in situ at a binding locus, direct insertion, abstraction-coupling or addition reactions will then result in covalent attachment of label to the site.

See "Nitrenes" (W. Lwowski, ed., Wiley Interscience, New York, 1970); T. L. Gilchrist and C. W. Rees. "Carbenes, Nitrenes and Arynes," Nelson, London, 1969.

In a third embodiments, the PF∗ is a free radical. The precursor is preferably an alpha,beta-unsaturated ketone. Free radicals are efficient hydrogen abstractors with a preference for carbon-hydrogen bonds; they are less likely to react with water molecules in the media.

While the PF∗ is preferably a carbene, nitrene or free radical, it may be another photogenerated, highly reactive species which reacts with biomolecules, especially those containing amino acid or sugar units. It is desirable that the PF∗ react not only with cysteine, lysine, tyrosine and tryptophan, but also with other amino acids or chemical moieties.

The reactivity of the PF∗ may in some cases be improved. Thus, electron-withdrawing substituents such as nitro groups enhance the reactivity of arylnitrenes. The electron-withdrawing power of a substitutent is indicated by its inductive substituent constant, see, e.g., Fuson, Reactions of Organic Compounds 11-12 (1962). The value for the nitro group is 0.78.

Other properties of the BFCA may also be modified. A nitro substituent may be used to shift the UV absorption spectrum of an arylazide to longer wavelengths. A trifluoromethyl function may be used to stabilize a diazoacetyl group. Hydrophilic substituents may be used to improve the solubility of the BFCA in aqueous media.

## Example 1: Synthesis of para-Azidobenzyl ethylene diamine tetra acetic acid (AzBEDTA)

This compound was chosen as the most suitable example of this class of photoactive BFCA (PF-BFCA) due to our experience in the synthesis of EDTA derivatives, the exceptional stability of metal-ion EDTA complexes for in vitro and in vivo use as well as the advantages of aryl azides as photoactive functionalities. All synthesis was accomplished using reagent grade chemicals and double distilled deionized water.

The stock precursor para-aminobenzylethylenediaminetetraacetic acid $\underline{1}$ was synthesized as described by Yeh, et al., Anal. Biochem., 100: 152 (1979). This material was converted to the diazonium intermediate $\underline{2}$ using acidic sodium nitrite. To a stirred suspension of $\underline{1}$ (19.36 mg, 0.048 mmol) in 250 µl of 12.2 M HCl at 0°C was added 125 µl of 0.5 M $NaNO_2$ (0.0625 mmol) and the mixture stirred for 1 h. At this time excess nitrous acid was destroyed by adding 5 mg of urea with continuous stirring for an additional 20 min. The product was assayed by reacting aliquots with resorcinol to form a colored complex which can be monitored spectrophotometrically at 385 nm. Using an extinction coefficient of 21500 $M^{-1}$ $cm^{-1}$, the overall diazonium yield was estimated to be ~75%. This intermediate was converted to the azido analogue $\underline{3}$ by treatment with sodium azide. To the crude solution of $\underline{2}$ was added 35 µl of 2.86 M $NaN_3$ (6.5 mg, 0.1 mmol) in water with vigorous stirring for 1 h at 0°C. This acidic solution was carefully neutralized with NaOH and concentrated as the sodium salt by freeze-drying. The resulting powder was dissolved in water and loaded onto a 0.8 x 15 cm P-2 Biogel (Bio-Rad) column. Fractions were eluted using water and analyzed for the final compound using TLC (Rf 0.75; cellulose plates developed in acetonitrile 7:3 water). The appropriate fractions were pooled and freeze-dried to give an overall yield of ~70%. The purified compound was analyzed by IR and NMR as shown in Figure 4 and Figure 5 respectively. The resulting spectra yielded the following characterization of para-azidobenzylethylenediaminetetraacetic acid (sodium salt).

IR(KBr): 3386 $cm^{-1}$ (-OH); 2122 $cm^{-1}$ (-$N_3$)

$^1$H-NMR (300 MHz, $D_2O$,pH-12): 7040 (d, 2H, J=9Hz); 6.880 (d, 2H, J=9Hz); 3.240 (d, 1H, J=16Hz); 3.090 (m, 1H); 2.905 (d, 1H, J=16Hz); 2.763 (d, 1H, J=16Hz); 2.692 (d, 1H, J=16Hz); 2.683 (d, 1H, J=16Hz); 2.625 (d, 1H, J=16Hz); 2.443 (d, 1H, J=16Hz); 2.312 (t, 2H, J=14Hz); 2.271 (d, 1H, J=16Hz); 2.194 (d, 1H, J=12Hz); 2.156 (d, 1H, J=12Hz).

The title compound was stable in the dark at 4°C for at least 4 weeks.

## Example 2: Chelation of Radioisotope by AzBEDTA -

One of the most useful radioactive metal ions currently used for radioimmunoimaging studies is Indium-111 (In-111) and therefore this tracer was chosen for the binding studies to make them relevant to current methods. To asses the degree of conjugation we contained simple assay procedures with standard radioisotope tracer methodology.

The chelation of In-111 by AzBEDTA was accomplished by adding a radiochemical grade (no-carrier-added) solution (10-100 µl) of In-111 in 0.05 N HCl (Atomic Energy of Canada Ltd.) to a solution (20-200 µl) of AzBEDTA (0.05 mmol) in 0.1 M citrate and reacting at room temperature for 30 min. Quantitative conversion to AzBEDTA-In-111 takes place as indicated by TLC analysis (acetonitrile: water 7:3, cellulose) in which free In-111 remains at the origin and the chelate migrates with a Rf 0.7.

Example 3: Photolysis of [In [111]] - AzBEDTA and Conjugation to Protein

Photolysis apparatus and procedure

The radiolabelled AzBEDTA (AzBEDTA-In-111) was used to determine the extent of binding via the photoactive group to bovine serum albumin (BSA) under various conditions. BSA is a common laboratory standard for numerous procedures requiring proteinaceous material. Stock solutions of both the reactants of varying concentrations in several buffers were prepared and the required volumes mixed just prior to irradiation.

The photolysis apparatus is a Hanoria UV lamp (254 nm) in a quartz jacketed cell of 1 cm path length through which ice water is rapidly circulated. A number of samples can be irradiated concurrently by mixing appropriate amounts of the radiolabelled chelate and protein in the required buffers in a quartz tube (1.2 cm x 7 cm) and clamping them in such a fashion that the irradiation of the reaction mixture takes place at a distance of 10 cm from the center of the lamp. The whole setup is placed in a hollow container so that the photolysis experiment can be done safely on the workbench itself. Thus, varying ratios of AzBEDTA-In-111 are added to BSA (as a 5% solution in the appropriate buffer) and the samples photolyzed for various time intervals (1-10 min.)

The degree of conjugation was determined by a simple TLC assay. An aliquot of the photolyzed reaction mixture was spotted on cellulose plates (0.8 x 8 cm) and developed in 0.1 molar HCl:methanol (3:7). In this system protein-bound AzBEDTA-In-111 remains at the origin and AzBEDTA-In-111 migrates with an Rf approximately 0.7. Since both species contain a radioactive isotope, the percentage in each fraction was calculated from measurements of the radioactivity from each section of the TLC plate. The number of chelates bound per molecule of protein is then determined from the initial molar ratio of reactants.

The factors affecting the performance of the new PF-BFCA were evaluated in a series of simple experiments. There appears to be little influence of protein concentration on the reaction as shown in Table 1. The reaction volume seems to be important as shown in Table 2, which may be indicative of some aspect of the light transfer process. When the concentration of the PF-BFCA is increased relative to the protein it appears to be more efficient process (Table 3). It must be compared that the actual nuclear of chelates bound is a function of both reaction yield and the initial ratio so the 18% of a 1:10 reaction results in about 2 chelates bound per protein molecule compared to 80% of 1:100 to give 80 chelates per molecule. As shown in Table 4, the duration of irradiation shows a distinct positive correlation with the binding. As expected, the degree of photoactivation is related to the total UV and visible light energy input into the system. The binding can also be influenced by the pH of the aqueous media in which the reaction takes place. In basic solutions the reaction is quantitative (Table 5). This may be explained by the propensity of the aryl nitrene to abstract a hydrogen atom, which is facilitated by alkaline conditions.

These studies demonstrate that under readily defined conditions the reaction between $^{111}$In-AzBEDTA and BSA can produce quantitative binding of the chelate to the protein. To verify this, BSA was conjugated under identical conditions with non-radioactive AzBEDTA and $^{111}$In citrate added after photolysis. The analysis of this solution demonstrated $^{111}$In binding to the protein via the second protocol (i.e., the pre-chelation of protein using photolysis of photoactive BFCA (PF-BFCA) in the presence of protein and then addition of metal ion to the purified protein) for metal-ion attachment using BFCA and substantiated the presence of chelating groups on the AzBEDTA treated BSA.

Example 4: Photolysis of a Monoclonal Antibody

To demonstrate that the reaction conditions for photoactivation of the chelate should not influence the biological performance of biomolecules we have used a monoclonal antibody (MAb-155) in the photolysis apparatus and measured its relative Immunoreactivity after the procedure. The ELISA technique was used to determine the binding efficiency of MAb-155 at various concentrations to an immobilized antigen. As shown in Table 6 there is no apparent change in the relative antigen binding of MAb-155 after 5 min of photolysis compared to MAb-155 alone or in the presence of the chelate. Therefore by the determination of immunological function, MAb-155 is unaffected by the process and retains its reactivity to antigenic material.

## TABLE 1

| Parameter | Value | % Bound to Protein |
|---|---|---|
| Protein:Ph-BFCA | 1:10 | - |
| Protein concentration | 2 mg/ml | 14 |
| | 5 mg/ml | 18 |
| | 10 mg/ml | 15 |
| Buffer (pH) | 0.1 M citrate (6.1) | - |
| Total reaction volume | 0.2 ml | - |
| Photolysis duration | 5.0 min | - |

## TABLE 2

| Parameter | Value | % Bound to Protein |
|---|---|---|
| Protein:Ph-BFCA | 1:100 | - |
| Protein concentration | 5 mg/ml | - |
| Buffer (pH) | 0.1 M citrate (6.1) | - |
| Total reaction volume | 0.2 ml | 80 |
| | 0.5 ml | 24 |
| Photolysis duration | 5.0 min | - |

## TABLE 3

| Parameter | Value | % Bound to Protein |
|---|---|---|
| Protein:Ph-BFCA | 1:1 | 34 |
| | 1:10 | 18 |
| | 1:100 | 80 |
| Protein concentration | 5 mg/ml | - |
| Buffer (pH) | 0.1 M citrate (6.1) | - |
| Total reaction volume | 0.2 ml | - |
| Photolysis duration | 5.0 min | - |

## TABLE 4

| Parameter | Value | % Bound to Protein |
|---|---|---|
| Protein:Ph-BFCA | 1:10 | - |
| Protein concentration | 10 mg/ml | - |
| Buffer (pH) | 0.1 M citrate (6.1) | - |
| Total reaction volume | 0.2 ml | - |
| Photolysis duration | 1.0 min | 4 |
| | 3.0 min | 7 |
| | 5.0 min | 15 |
| | 10.0 min | 100 |

## TABLE 5

| Parameter | Value | % Bound to Protein |
|---|---|---|
| Protein:Ph-BFCA | 1:10 | - |
| Protein concentration | 10 mg/ml | - |
| Buffer (pH) | 2.0 M phosphate (1.5) | 7 |
| | 0.1 M citrate (6.1) | 15 |
| | 0.2 M phosphate (6.2) | 17 |
| | 0.5 M phosphate (7.8) | 99 |
| | 0.5 M phosphate (9.0) | 99 |
| Total reaction volume | 0.2 ml | - |
| Photolysis duration | 5.0 min | - |

## TABLE 6

| Micrograms of MAb-155 per well | Optical Density Value In Standardized ELISA | | |
|---|---|---|---|
| | MAb-155 | MAb-155 +100:1 AzBEDTA | MAb-155 +100:1 AzBEDTA +5 min photolysis |
| 10 | $0.81 \pm 0.01$ | $0.78 \pm 0.04$ | $0.82 \pm 0.08$ |
| 3 | $0.82 \pm 0.05$ | $0.79 \pm 0.07$ | $0.85 \pm 0.10$ |
| 1 | $0.78 \pm 0.04$ | $0.74 \pm 0.03$ | $0.83 \pm 0.07$ |
| 0.1 | $0.53 \pm 0.06$ | $0.47 \pm 0.02$ | $0.68 \pm 0.09$ |
| 0.01 | $0.12 \pm 0.01$ | $0.12 \pm 0.02$ | $0.22 \pm 0.05$ |

$x \pm s.d.$; $n - 3$.

**Claims**

1. A composition comprising a compound having a chelating group and a photoactivatable group which upon photoactivation is capable of reaction with a carbon-hydrogen, nitrogen-hydrogen, or oxygen-hydrogen bond.

2. The composition of claim 1 wherein the photoactivatable group is photoactivatable to become a carbene.

3. The composition of claim 1 wherein the photoactivatable group is photoactivatable to become a nitrene.

4. The composition of claim 1 wherein the photoactivatable group is photoactivatable to become a free radical.

**5.** The composition of claim 3 wherein the photoactivatable group is an arylazide.

**6.** The composition of claim 2 wherein the photoactivatable group is an aryldiazarine.

**7.** The composition of claim 2 wherein the photoactivatable group is a fluoroalkyl diazoacetyl group.

**8.** A method of conjugating a chelatable ion with a biomolecule which comprises in any order the steps of (a) photoactivating the compound of claim 1 in the presence of a biomolecule, whereby the photoactivated group is coupled to the biomolecule, and (b) incubating a chelatable ion with said compound under chelating conditions.

**9.** The method of claim 8 wherein step (b) precedes or is simultaneous with step (a).

**10.** The method of claim 8 wherein step (b) follows step (a).

**11.** The method of claim 8 wherein the photoactivatable group is photoactivated to become a carbene.

**12.** The method of claim 8 wherein the photoactivatable group is photoactivated to become a nitrene.

**13.** The method of claim 8 wherein the photoactivatable group is photoactivated to become a free radical.

**14.** The method of claim 12 wherein the photoactivatable group is an arylazide.

**15.** The method of claim 11 wherein the photoactivatable group is an aryldiazarine.

**16.** The method of claim 11 wherein the photoactivatable group is a fluoroalkyl diazoacetyl group.

**17.** The composition of claim 1 wherein a chelatable ion is chelated by the chelating group.

**18.** The method of claim 8 wherein the biomolecule is an antibody.

**Patentansprüche**

**1.** Zusammensetzung, umfassend eine Verbindung mit einer chelatbildenden Gruppe und einer fotoaktivierbaren Gruppe, die bei Fotoaktivierung eine Reaktion mit einer Kohlenstoff-Wasserstoff-, Stickstoff-Wasserstoff- oder Sauerstoff-Wasserstoff-Bindung eingehen kann.

**2.** Zusammensetzung nach Anspruch 1, worin die fotoaktivierbare Gruppe so fotoaktivierbar ist, daß sie zu einem Carben wird.

**3.** Zusammensetzung nach Anspruch 1, worin die fotoaktivierbare Gruppe so fotoaktivierbar ist, daß sie zu einem Nitren wird.

**4.** Zusammensetzung nach Anspruch 1, worin die fotoaktivierbare Gruppe so fotoaktivierbar ist, daß sie zu einem freien Radikal wird.

**5.** Zusammensetzung nach Anspruch 3, worin die fotoaktivierbare Gruppe ein Arylazid ist.

**6.** Zusammensetzung nach Anspruch 2, worin die fotoaktivierbare Gruppe ein Aryldiazarin ist.

**7.** Zusammensetzung nach Anspruch 2, worin die fotoaktivierbare Gruppe eine Fluoralkyldiazoacetyl-Gruppe ist.

**8.** Verfahren zur Konjugation eines chelatisierbaren Ions mit einem Biolmolekül, umfassend die folgenden Schritten in beliebiger Reihenfolge: (a) Fotoaktivierung der Verbindung nach Anspruch 1 in Anwesenheit eines Biomoleküls, wodurch die fotoaktivierte Gruppe mit dem Biomolekül gekuppelt wird, und (b) Einführen eines chelatisierbaren Ions in diese Verbindung unter chelatisierenden Bedingungen.

**9.** Verfahren nach Anspruch 8, worin Schritt (b) vor oder gleichzeitig mit dem Schritt (a) durchgeführt wird.

10. Verfahren nach Anspruch 8, worin Schritt (b) Schritt (a) folgt.

11. Verfahren nach Anspruch 8, worin die fotoaktivierbare Gruppe fotoaktiviert wird, um ein Carben zu werden.

12. Verfahren nach Anspruch 8, worin die fotoaktivierbare Gruppe fotoaktiviert wird, um ein Nitren zu werden.

13. Verfahren nach Anspruch 8, worin die fotoaktivierbare Gruppe fotoaktiviert wird, um ein freies Radikal zu werden.

14. Verfahren nach Anspruch 12, worin die fotoaktivierbare Gruppe ein Arylazid ist

15. Verfahren nach Anspruch 11, worin die fotoaktivierbare Gruppe ein Aryldiazarin ist.

16. Verfahren nach Anspruch 11, worin die fotoaktivierbare Gruppe eine Fluoralkyldiazoacetyl-Gruppe ist.

17. Zusammensetzung nach Anspruch 1, worin ein chelatisierbares Ion durch die chelatisierenden Gruppe chelatisiert ist.

18. Verfahren nach Anspruch 8, worin das Biomolekül ein Antikörper ist.

**Revendications**

1. Composition comprenant un composé qui comporte un groupe chélatant et un groupe photoactivable qui, après phtotactivation, est à même de subir une réaction avec une liaison carbone-hydrogène, azote-hydrogène ou oxygène-hydrogène.

2. Composition selon la revendication 1, dans laquelle le groupe photoactivable est photoactivable de façon à devenir un carbène.

3. Composition selon la revendication 1, dans laquelle le groupe photoactivable est photoactivable pour devenir un nitrène.

4. Composition selon la revendication 1, dans laquelle le groupe photoactivable est photoactivable pour devenir un radical libre.

5. Composition selon la revendication 3, dans laquelle le groupe photoactivable est un arylazide.

6. Composition selon la revendication 2, dans laquelle le groupe photoactivable est une aryldiazarine.

7. Composition selon la revendication 2, dans laquelle le groupe photoactivable est un groupe fluoroalkyldiazoacétyle.

8. Procédé de conjugaison d'un ion chélatable et d'une biomolécule, qui comprend, dans un ordre quelconque, les étapes consistant (a) à photoactiver le composé selon la revendication 1 en présence d'une biomolecule, le groupe photoactivé étant ainsi couplé à la biomolécule, et (b) à incuber l'ion chélatable avec ledit composé dans les conditions d'une chélation.

9. Procédé selon la revendication 8, dans lequel l'étape (b) précède l'étape (a) ou lui est simultanée.

10. Procédé selon la revendication 8, dans lequel l'étape (b) suit l'étape (a).

11. Procédé selon la revendication 8, dans lequel le groupe photoactivable est photoactivable de façon à devenir un carbène.

12. Procédé selon la revendication 8, dans lequel le groupe photoactivable est photoactivable pour devenir un nitrène.

13. Procédé selon la revendication 8, dans lequel le groupe photoactivable est photoactivable pour devenir un radical libre.

**14.** Procédé selon la revendication 8, dans lequel le groupe photoactivable est un arylazide.

**15.** Procédé selon la revendication 8, dans lequel le groupe photoactivable est une aryldiazarine.

**16.** Procédé selon la revendication 8, dans lequel le groupe photoactivable est un groupe fluoroalkyldiazoacétyle.

**17.** Composition selon la revendication 1, dans laquelle un ion chélatable est chélaté par le groupe chélatant.

**18.** Procédé selon la revendication 8, dans lequel la biomolécule est un anticorps.